# EUROPEAN PATENT APPLICATION

(11) **EP 4 726 509 A1**
(43) Date of publication of application: **15.04.2026**
(21) Application number: 25207717.7
(22) Date of filing: 09.10.2025
(51) Int. Cl.: G06F 3/01, G02B 27/00

(54) **AN EYE-TRACKING SYSTEM AND METHODS OF USING AN EYETRACKING SYSTEM**

(30) Priority: 10.10.2024 SE 2451007
(71) Applicant: Tobii AB, 182 17 Danderyd (SE)
(72) Inventor: Blixt, Peter, 12938 Hägersten (SE); Johansson Tornéus, Daniel, 171 69 Solna (SE); Tirona Remulla, Katherine Isabel, 11421 Stockholm (SE)
(74) Representative: Borodulina, Ellie

(57) **Abstract**

An eye-tracking system for tracking an eye that includes a holographic optical element (HOE). The HOE has one or more specularly reflective regions and one or more diffusely reflective regions that are provided as a predetermined pattern. the eye-tracking system also includes a camera and a controller. The camera is configured to capture an image of the eye as reflected by the one or more specularly reflective regions of the holographic optical element, such that a pattern that is defined by the one or more diffusely reflective regions of the holographic optical element is visible on the eye in the captured image and the one or more diffusely reflective regions. The controller is configured to: process the captured image of the eye in order to recognise thea pattern of the one or more diffusely reflective regions in the image; and compare: i) the predetermined pattern, with ii) the recognised pattern in the captured image, in order to determine a distance from the holographic optical element to the eye.

## Description

### Field

The present disclosure generally relates to the field of eye tracking. In particular, the present disclosure relates to methods and systems for determining a distance between an eye and a holographic optical element in an eye-tracking system.

### Background

In eye tracking applications, digital images are retrieved of the eyes of a user and the digital images are analysed in order to estimate gaze direction of the user. The estimation of the gaze direction may be based on computer-based image analysis of features of the imaged eye. One known example method of eye tracking includes the use of infrared light and an image sensor. The infrared light is directed towards eye(s) of a user and the reflection of the light is captured by an image sensor.

Portable or wearable eye-tracking devices have been previously described. One such eye-tracking system is described in U.S. Pat. No. 9,041,787 and PCT patent publication number WO 2019/158709 (which are hereby incorporated by reference in their entirety). A wearable eye-tracking device is described using illuminators and cameras for determining gaze direction.

### Summary

According to a first aspect of the disclosure, there is provided an eye-tracking system for tracking an eye, the eye-tracking system comprising:
a holographic optical element, which comprises one or more specularly reflective regions and one or more diffusely reflective regions, wherein the one or more specularly reflective regions and the one or more diffusely reflective regions of the holographic optical element are provided as a predetermined pattern;
a camera that is configured to capture an image of the eye as reflected by the one or more specularly reflective regions of the holographic optical element, such that a pattern that is defined by the one or more diffusely reflective regions of the holographic optical element is visible on the eye in the captured image; and
a controller that is configured to:
   process the captured image of the eye in order to recognise the pattern of the one or more diffusely reflective regions in the image; and
   compare: i) the predetermined pattern, with ii) the recognised pattern in the captured image, in order to determine a distance from the holographic optical element to the eye.

The controller may be configured to determine a plurality of distances from the holographic optical element to respective different regions of the eye.

The controller may be configured to determine a corneal topography map based on the determined plurality of distances.

The predetermined pattern may comprise a 2-dimensional pattern with a predetermined spacing between features in the pattern in two perpendicular dimensions.

The controller may be configured to:
compare: i) the predetermined spacing between features in the predetermined pattern, with ii) a recognised spacing between corresponding features in the recognised pattern in the captured image in order to:
determine a distance from the holographic optical element to regions of the eye on which the corresponding features in the recognised pattern in the captured image are present.

The controller may be configured to:
compare: i) the predetermined pattern, with ii) the recognised pattern in the captured image, in order to:
detect any distortions in the recognised pattern; and
determine the distance from the holographic optical element to the eye based on the detected distortions.

The camera may be configured to capture a plurality of images of the eye as reflected by the one or more specularly reflective regions of the holographic optical element, at a respective plurality of difference instants in time, such that a pattern that is defined by the one or more diffusely reflective regions of the holographic optical element is visible on the eye in each of the captured images.

The controller may be configured to, for each of the plurality of captured images:
process the captured image of the eye in order to recognise the pattern in the image of the one or more diffusely reflective regions; and
compare: i) the predetermined pattern, with ii) the recognised pattern in the captured image, in order to extract a portion of the captured image in which the recognised pattern is present;

The controller may further be configured to:
combine the extracted portions of the captured images to provide a combined-image of the eye including a plurality of recognised patterns; and
compare each of the plurality of recognised patterns in the combined-image with the predetermined pattern in order to determine a distance between: i) the regions of the eye that are represented by the recognised patterns; and ii) the holographic optical element.

The predetermined pattern may comprise a substantially one-dimensional pattern.

The controller may be further configured to:
update an eye-tracking model based on the determined distance from the holographic optical element to the eye; and
use the updated eye-tracking model to perform eye-tracking based on subsequently captured images of the eye.

During a subsequent eye-tracking operation, the illuminator may be configured to:
provide background illumination of the eye by illuminating the one or more diffusely reflective regions of the holographical optical element; and
provide one or more "glints" on the eye by illuminating the one or more specularly reflective regions of the holographical optical element.

The holographic optical element may comprise a plurality of specularly reflective regions, such that the illuminator is configured to provide a plurality of "glints" on the eye by illuminating the plurality of specularly reflective regions of the holographical optical element.

The eye-tracking system may comprise a head-mounted device.

The head-mounted device may comprise a pair of glasses having lens regions and a pair of arms, and wherein:
the camera may be mounted to one of the arms; and / or
the holographic optical element may be located over at least one of the lens regions.

The eye-tracking system may further comprise an illuminator that is configured to illuminate the eye via the one or more specularly reflective regions and the one or more diffusely reflective regions of the holographic optical element.

There is also disclosed a computer implemented method of operating an eye-tracking system, wherein the eye-tracking system comprises:
a holographic optical element, which comprises one or more specularly reflective regions and one or more diffusely reflective regions, wherein the one or more specularly reflective regions and the one or more diffusely reflective regions of the holographic optical element are provided as a predetermined pattern;
the method comprising:
   capturing an image of the eye as reflected by the one or more specularly reflective regions of the holographic optical element, such that a pattern that is defined by the one or more diffusely reflective regions of the holographic optical element is visible on the eye in the captured image; and
   processing the captured image of the eye in order to recognise the pattern of the one or more diffusely reflective regions in the image; and
   comparing: i) the predetermined pattern, with ii) the recognised pattern in the captured image, in order to determine a distance from the holographic optical element to the eye.

There may be provided a computer program, which when run on a computer, causes the computer to configure any apparatus, including a controller, device or system disclosed herein or perform any method disclosed herein. The computer program may be a software implementation, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be an assembly program.

The computer program may be provided on a computer readable medium, which may be a physical computer readable medium such as a disc or a memory device, or may be embodied as a transient signal. Such a transient signal may be a network download, including an internet download. There may be provided one or more non-transitory computer-readable storage media storing computer-executable instructions that, when executed by a computing system, causes the computing system to perform any method disclosed herein.

### Brief Description of the Drawings

One or more embodiments will now be described by way of example only with reference to the accompanying drawings in which:
Figure 1 shows a simplified view of a head-mounted eye-tracking system;
Figure 2 shows a simplified example of an image of a pair of eyes, captured by an eye-tracking system such as the system of Figure 1;
Figure 3 shows an example head-mounted eye-tracking system according to an embodiment of the present disclosure;
Figure 4a shows an example set-up that can be used to provide a holographic optical element (HOE) that has a predetermined pattern of specularly reflective regions and diffusely reflective regions;
Figure 4b shows an example of a patterned object that is used in the set-up of Figure 4a to create a patterned HOE;
Figure 5 shows an example of an image of an eye that has been captured by a camera via an HOE with a concentric ring pattern of specularly and diffusely reflective regions;
Figure 6 shows another example of an image of an eye that has been captured by a camera via an HOE with a concentric ring pattern of specularly and diffusely reflective regions; and
Figure 7 shows a flow chart that illustrates a method of operating an eye-tracking system according to the present disclosure.

### Detailed Description

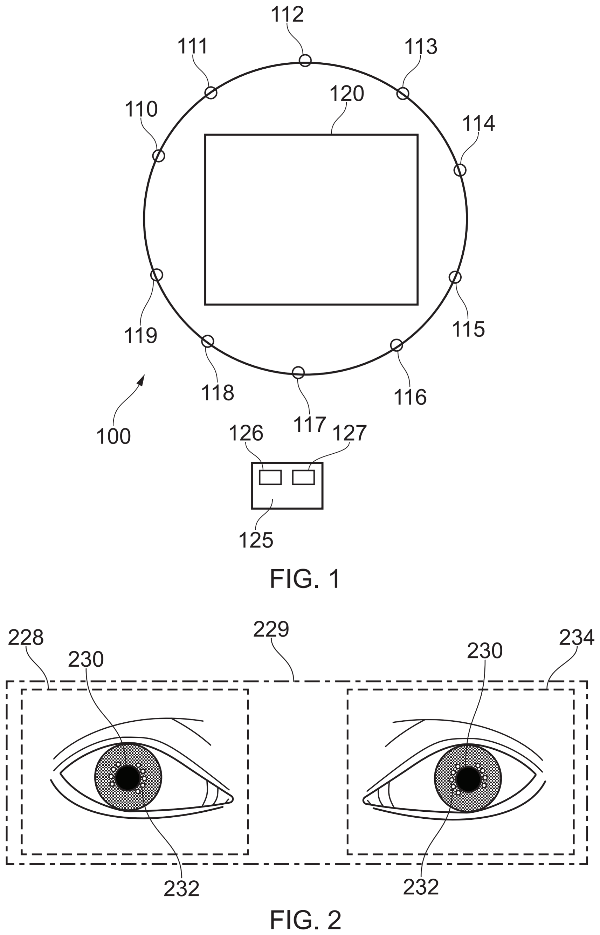

Figure 1 shows a simplified view of a head-mounted eye-tracking system 100 (which may also be referred to as a gaze tracking system) in the form of a virtual or augmented reality (VR or AR) device or VR or AR glasses or anything related, such as extended reality (XR) or mixed reality (MR) headsets. The system 100 comprises an image sensor 120 (e.g., a camera) for capturing images of the eyes of the user. The system may optionally include one or more illuminators 110-119 (also referred to herein as light sources) for illuminating the eyes of a user, which may for example be light emitting diodes (LEDs) emitting light in the infrared frequency band, or in the near infrared frequency band and which may be physically arranged in a variety of configurations. The image sensor 120 may for example be an image sensor of any type, such as a complementary metal oxide semiconductor (CMOS) image sensor or a charged coupled device (CCD) image sensor. The image sensor may consist of an integrated circuit containing an array of pixel sensors, each pixel containing a photodetector and an active amplifier. The image sensor may be capable of converting light into digital signals. In one or more examples, it could be an infrared image sensor or IR image sensor, an RGB sensor, an RGBW sensor or an RGB or RGBW sensor with IR filter.

The eye-tracking system 100 may comprise circuitry or one or more controllers 125, for example including a receiver 126 and processing circuitry 127, for receiving and processing the images captured by the image sensor 120. The circuitry 125 may for example be connected to the image sensor 120 and the optional one or more illuminators 110-119 via a wired or a wireless connection and be co-located with the image sensor 120 and the one or more illuminators 110-119 or located at a distance, e.g., in a different device. In another example, the circuitry 125 may be provided in one or more stacked layers below the light sensitive surface of the light sensor 120.

The eye-tracking system 100 may include a display (not shown) for presenting information and/or visual stimuli to the user. The display may comprise a VR display which presents imagery and substantially blocks the user's view of the real-world or an AR display which presents imagery that is to be perceived as overlaid over the user's view of the real-world.

The location of the image sensor 120 for one eye in such a system 100 is generally away from the line of sight for the user in order not to obscure the display for that eye. This configuration may be, for example, enabled by means of so-called hot mirrors which reflect a portion of the light and allows the rest of the light to pass, e.g., infrared light is reflected, and visible light is allowed to pass.

While in the above example the images of the user's eye are captured by a head-mounted image sensor 120, in other examples the images may be captured by an image sensor that is not head-mounted. Such a non-head-mounted system may be referred to as a remote system.

Figure 2 shows a simplified example of an image 229 of a pair of eyes, captured by an eye-tracking system such as the system of Figure 1. The image 229 can be considered as including a right-eye-image 228, of a person's right eye, and a left-eye-image 234, of the person's left eye. In this example the right-eye-image 228 and the left-eye-image 234 are both parts of a larger image of both of the person's eyes. In other examples, separate image sensors may be used to acquire the right-eye-image 228 and the left-eye-image 234. In further still examples, multiple image sensors may be used to acquire images capturing both eyes.

The system may employ image processing (such as digital image processing) for extracting features in the image. The system may for example identify a position of the pupil 230 in the one or more images captured by the image sensor. The system may determine the position of the pupil 230 using a pupil detection process. The system may also identify corneal reflections (also known as glints) 232 located in close proximity to the pupil 230. The system may estimate a corneal centre and / or a distance to the user's eye based on the corneal reflections 232. For example, the system may match each of the individual corneal reflections 232 for each eye with a corresponding illuminator and determine the corneal centre of each eye and / or the distance to the user's eye based on the matching. To a first approximation, the eye-tracking system may determine an optical axis of the eye of the user as the vector passing through a centre of the pupil 230 and the corneal centre. The direction of gaze corresponds to the axis from the fovea of the eye through the corneal centre (visual axis). The angle between the optical axis and the gaze direction is the *foveal offset,* which typically varies from user to user and is in the range of a few degrees. The eye-tracking system may perform a calibration procedure, instructing the user to gaze in a series of predetermined directions (e.g., via instructions on a screen), to determine the fovea offset. The determination of the optical axis described above is known to those skilled in the art and often referred to as pupil centre corneal reflection (PCCR). PCCR is not discussed in further detail here.

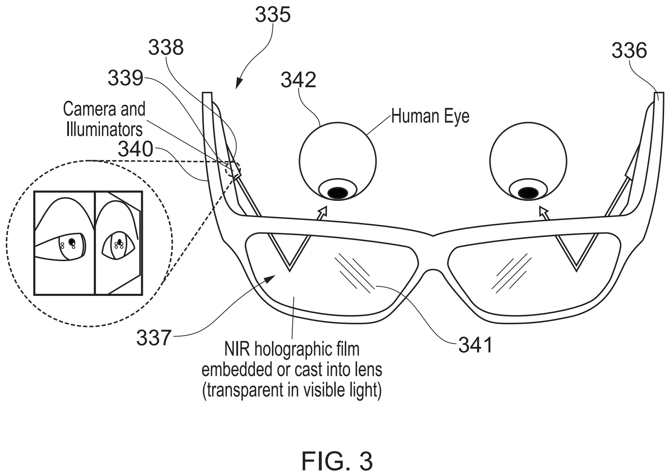

Figure 3 shows an example head-mounted eye-tracking system 335 according to an embodiment of the present disclosure. As will be discussed below, this head-mounted eye-tracking system 335 is a holographic eye-tracking system.

In this example, the eye-tracking system 335 includes a pair of glasses 336 that has two lens regions 337 (one for each eye 342) and a pair of arms 340. In addition, an illuminator 339 is mounted to each of the arms 340 such that each eye is individually illuminated. Also in this example, a camera 338 is mounted to each of the respective arms 340 such that an image of each eye 342 can be individually captured. As shown in Figure 3, each illuminator 339 and camera 338 are located close to each other on their respective arms 340. Although not shown in Figure 3, the eye-tracking system 335 also includes a controller for processing the captured images. The controller may be located on the pair of glasses 336 or it may be located remote from the pair of glasses 336. Either way, it is in electronic communication with at least the camera 338.

A holographic optical element (HOE) 341 is located over each of the of the respective lens regions 337. The HOEs 341 can be implemented by embedding a near infra-red (NIR) holographic film over the lenses, or by casting a NIR holographic film into the lenses. Such NIR holographic films are invisible / transparent to visible light, and therefore they do not obscure the user's view through the lens regions 337. However, as will be discussed below, because the NIR holographic films are reflective for NIR light they can be used for eye tracking purposes.

In one example, at least parts of the HOE 341 are provided as specularly reflective regions. Furthermore, as known in the art, they can be provided such that they provide the functionality of one or more virtual mirrors that are oriented at an angle that is not necessarily co-planar with the lens region 337 on which the HOE 341 is embedded or in which it is cast. Such virtual mirrors are known in the art, for example as described in: Tomoya Nakamura, Shinji Kimura, Kazuhiko Takahashi, Yuji Aburakawa, Shunsuke Takahashi, Shunsuke Igarashi, Shiho Torashima, and Masahiro Yamaguchi, "Off-axis virtual-image display and camera by holographic mirror and blur compensation," Opt. Express 26, 24864-24880 (2018); and Nakamura T, Kimura S, Takahashi K, Aburakawa Y, Takahashi S, Igarashi S, et al. Holographic Pepper's Ghost: Upright Virtual-Image Screen Realized by Holographic Mirror [Internet]. Holographic Materials and Applications. IntechOpen; 2019. Available from: http://dx.doi.org/10.5772/intechopen.85600.

Eye tracking can be performed by the eye-tracking system 335 of Figure 3 by the illuminators 339 emitting light such that it is bounced off specularly reflective regions in the HOE 341 towards the eye 342. As is known in the art, and as identified briefly above, such light can create glints on the eye 342. Then, the cameras 338 can capture images of the eye 342, as reflected by a specularly reflective region of the HOE 341, that include those glints such that eye tracking can be performed. In this way, illumination is provided through the same virtual mirror that is used by the camera 338 to capture the images. Generally, the illuminator 339 can be placed close to the camera 338 (for example, within a few mm).

Alternatively, the illuminators for creating the glints can be located at different locations. For instance, the illuminators can be around the periphery of the HOE 341 such that they illuminate the eye directly. However, when illuminators are placed around the HOE, it can have a drawback that only a low percentage of the light illuminates the eye. Sometimes as low as 10%. In general, it can result in good glints at high gaze angles, but can struggle to create glints when the eye is looking straight ahead. At very low eye-relief and / or narrow eyelid opening there can be too few or no glints at all.

For illuminators 339 that bounce light off a virtual mirror in the HOE 341 (such as the ones shown in Figure 3), they can have a drawback of not utilizing all of the illumination if an insufficient amount of the entire hologram film is reflective, and therefore not all of the HOE 341 reflects light towards the eye 342. Furthermore, if all the lights on the eye 342 are from the specularly reflective regions (which can also be referred to as mirror portions) of the HOE 341, including those that create the glint reflection, then the glint-to-illumination contrast ratio can be very high. This can result in the glints being strongly saturated in the image, thereby making them larger, and potentially obfuscating eye features such as the pupil or iris. Furthermore, since the illumination is provided through a path between the illuminator 339 and the HOE 341 to the eye 342, it can be very sensitive to being blocked by the eyelid and eyelashes. As a result, there can be cases where the illumination of the pupil and iris could be poor due to the light being blocked.

In the examples disclosed herein, the HOE 341 includes one or more specularly reflective regions and one or more diffusely reflective regions. As discussed above, a specularly reflective region can provide the functionality of a virtual mirror such that the camera 338 can capture the image of the eye 341 via the HOE 341. It is not possible for the camera 338 to obtain accurate images of the eye 342 via the diffusely reflective regions, which reflect light in a similar way to that of white paper. However, the illuminators 339 in Figure 3, which illuminate the entire HOE 341, can provide background lighting to the eye 342 which is less intense than the light that is provided to the eye 342 via the specularly reflective regions in order to create the glints.

By utilizing the area of the HOE 341 that does not reflect an image of the volume that the eye can be in (i.e., the diffusely reflective regions), a different reflective area can be created that does not act like a normal mirror. Ideally, such diffusely reflective regions possess a Lambertian reflectance profile so that the illumination is even. These diffuse areas can assist in providing uniform illumination of the eye, and also illumination of regions of the eye that previously were not illuminated, such as areas blocked by eyelashes or an eyelid. Since a direct path from an illuminator via a holographic mirror can be blocked, having a large area illuminating the eye can result in light from several directions hitting each part of the eye and thereby reducing the likelihood that a region of the eye is not sufficiently illuminated. This is very important for robust eye-tracking over a large population, especially for users with narrow eyelid openings or if sub-optimal component placement of cameras and illuminators is used.

As will now be discussed, HOEs of the present disclosure have one or more specularly reflective regions and one or more diffusely reflective regions. Furthermore, the one or more specularly reflective regions and the one or more diffusely reflective regions of the holographic optical element are provided as a predetermined pattern. Use of such a predetermined pattern can advantageously be used to determine a distance from the HOE to the eye, as will be discussed in detail below. Further still, use of such a predetermined pattern can be used to determine the respective distances to different regions of the eye such that a corneal topography map can be generated. In such instances, it will be appreciated that it is not necessary to calculate the absolute distances from the HOE to each region of the eye; relative distances to the different regions of the eye will be sufficient. The calculation of such determined distances / corneal maps can be used to improve the accuracy of a subsequent eye-tracking operation. For example, an eye-tracking model, that is used for eye-tracking as is known in the art, can be updated based on the determined distance / corneal map. Then, the updated eye-tracking model can be used to perform eye tracking based on subsequently captured images of the eye more accurately than would otherwise be the case.

In the main example that follows, the predetermined pattern of the specularly reflective regions and the diffusely reflective regions is one of concentric rings. However, we will also describe alternative predetermined patterns that can be used.

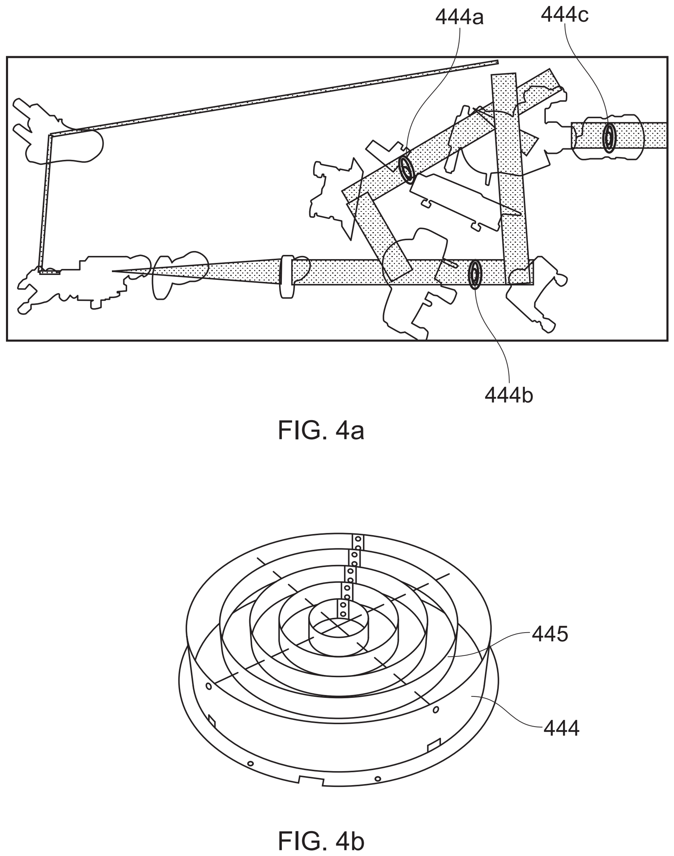

Figure 4a shows an example set-up that can be used to provide a HOE that has a predetermined pattern of specularly reflective regions and diffusely reflective regions. Figure 4b shows an example of a patterned object that is used in the set-up of Figure 4a to create the patterned HOE.

Figure 4a shows a set-up of a spherical beam interfering with two collimated beams. One of the collimated beams is provided with a patterned object, such as the object 444 of Figure 4b. As shown in Figure 4b, the patterned object 444 defines a pattern of concentric rings by having concentric light-blocking portions that define gaps therebetween through which light can pass. Example locations in the set-up of Figure 4a at which the patterned object 444 can be located are identified with reference numbers 444a, 444b, 444c. The rings defined by the patterned object 444 will be recorded simultaneously as the optical elements of mirror and lens. The concentric ring pattern can be recorded by having a physical pattern in the reference or object beam when recording the hologram. A flat mirror or a mirror with refraction having a spherical wave can be recorded in the other interference beam.

In this way, the HOE that is created by the set-up of Figure 4a is provided with a pattern of specularly and diffusely reflective regions, in this example of concentric rings. The pattern is generated by locating a physical shape (such as the patterned object 444) in one of the interference beams, preferably in a collimated beam. In the set-up of Figure 4a, there is a spherical beam for having a refractive lens effect recorded in the hologram, which can be beneficial for making telecentric imaging. There are two collimated beams in the set-up for making two holograms side-by-side. This can provide for a stereo view of the eye. The concentric ring pattern could be on only one hologram if the physical pattern is located in that beam.

It will be appreciated that the hologram creation technique of Figures 4a and 4b is not limiting. Any other known technique can be used, such as use of liquid crystals, amplitude modulation instead of phase modulation, etc.

Returning to Figure 3, we will now describe use of the eye-tracking system 335 with a patterned HOE 341. That is, an HOE 341 that has one or more specularly reflective regions and one or more diffusely reflective regions provided as a predetermined pattern. The pattern is described as predetermined in the sense that, when the controller comes to process the images of the eye 342 that have been captured via the HOE 341, it has details of the pattern that it is expecting to recognise in the image. The camera 338 captures an image of the eye 342 as reflected by the patterned HOE 341 such that a pattern that is defined by the one or more diffusely reflective regions of the holographic optical element is visible on the eye in the captured image.

The controller processes the captured image of the eye 342 in order to recognise the pattern of the one or more diffusely reflective regions in the image. The controller then compares: i) the predetermined pattern (details of which it can retrieve from computer memory, for example), with ii) the recognised pattern in the captured image. Then, based on the result of this comparison, it determines a distance from the HOE 341 to the eye 342. Various example implementations for determining such a distance are provided below.

The predetermined pattern is any pattern that has an arrangement of specularly and diffusely reflective regions that, when overlaid on the 3-dimensional surface of the eye (especially the cornea of the eye) is suitable for determining a distance from the HOE 341 to the eye 342. It will be appreciated from the specific examples that follow, as well as the skilled person's general knowledge, that a wide variety of predetermined patterns can be used and that the suitability of a predetermined pattern can easily be directly and positively verified by tests or procedures without undue experimentation. Such predetermined patterns can be considered as patterns with a known structure.

In some examples, the predetermined pattern is a 2-dimensional pattern, such as the concentric rings that are created by use of the patterned object of Figure 4b. The 2-dimensional pattern can have a predetermined spacing between features in the pattern. Such a predetermined spacing can be in one or both of two perpendicular dimensions of the 2-dimensional pattern. For the concentric ring example, the spacing between the features can be the spacing between pairs of rings. In an alternative example, the pattern can include sets of parallel lines (which could be straight or curved; horizontal, vertical or at any angle). The predetermined pattern can include one or more of: rings (that may or may not be concentric), arcs (that may or may not be concentric), crosses (where the intersections of the crosses are the features with a predetermined spacing therebetween). In yet further examples, the predetermined pattern may be a pattern of dots. In deciding what predetermined pattern to use for a given application, there can be a trade-off between how easy it is to find the pattern in the captured images and how capable it will be for determining distance to the eye (especially where multiple distances to different regions of the eye are determined for mapping the topography of the cornea, as will be discussed below). For example, a pattern that includes a cross can be easy to find, but it won't be able to provide as much topographical information as a dot pattern over a large proportion of the cornea. However, such a dot pattern can be more difficult to find in the captured image and more difficult to match with the corresponding parts of the predetermined pattern. Therefore, the most appropriate predetermined pattern can be different for different applications, depending upon what the calculated distance between the HOE and the eye is to be used for.

In examples of the present disclosure, the controller of the eye-tracking system 335 can compare: i) a predetermined spacing between features in the predetermined pattern, with ii) a recognised spacing between corresponding features in the recognised pattern in the captured image. Then, based on the result of the comparison, the controller can determine a distance from the holographic optical element to regions of the eye on which the corresponding features in the recognised pattern in the captured image are present. In the concentric rings example, the spacing between adjacent rings of the same type (i.e., rings as reflected by the specularly reflective regions, or rings as reflected by the diffusely reflective regions) will represent the distance between the HOE 341 and the eye. That is, the greater the distance between the HOE 341 and the eye 342, the greater the spacing between adjacent rings of the same type in the captured image. It is worth mentioning that, as will be appreciated from Figure 3, the camera 338 in this example is at a fixed distance with respect to the HOE 341. Therefore, there is no prospect for a change in distance between the camera 338 and the HOE 341 affecting the spacing between adjacent rings of the same type in the captured image.

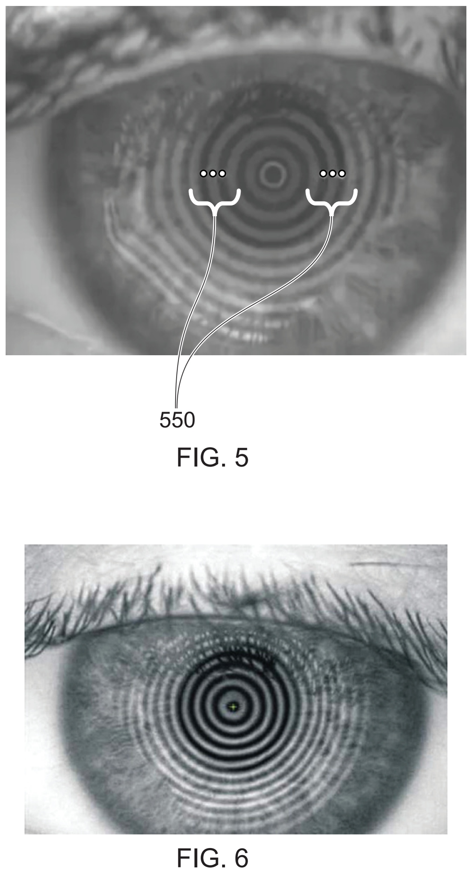

Figure 5 shows an example of an image of an eye that has been captured by a camera via an HOE with a concentric ring pattern of specularly and diffusely reflective regions.

It will be appreciated that the controller of Figure 4 can determine an absolute distance to one or more regions of the eye over which the recognised pattern is present. For instance, by applying an algorithm to the spacing between rings (which can also be considered as the thickness of a ring) that translates the spacing to an absolute distance (for example, in millimetres) between the HOE and the eye. The parameters of such an algorithm can be determined in a calibration operation, for example.

In examples where a plurality of distances to different regions of the eye are calculated, these can be used for corneal topography mapping of the eye. As will be discussed below, such a map can be especially useful for improving the accuracy of a subsequent eye tracking operation.

The controller of Figure 4 can, alternatively or additionally, determine a plurality of relative distances to different regions of the eye over which the recognised pattern is present. For example, relative variations of the thicknesses of rings in the concentric pattern will represent relative variations in the distances to the different regions of the eye on which the recognised pattern is present (assuming, of course, that the thicknesses of the concentric rings in the predetermined pattern in the HOE are consistent).

Returning to Figure 3, in some examples the controller can compare: i) the predetermined pattern, with ii) the recognised pattern in the captured image, in order to detect any distortions in the recognised pattern. The controller can then determine the distance from the HOE 341 to the eye 342 based on the detected distortions. For example, the controller can determine relative distances from the HOE 341 to the eye 342 for a plurality of regions of the eye 342 based on the detected distortions. This can be implemented by the controller applying any suitable mathematical operations to the detected distortions to convert them to a 3D corneal topography profile, for instance.

In particular examples of the present disclosure, a predetermined pattern is one that results in, when the camera captures the image of the eye via the specularly reflective regions of the HOE 341, a captured image that is suitable for determining the topography of the cornea of the eye.

The above examples of a predetermined pattern are 2-dimensional patterns. We will now describe an example that uses a 1-dimensional or a 2-dimensional pattern for determining the distance between the HOE 341 and a region of the eye 342. Such examples can beneficially utilise the HOE 341 to perform laser line triangulation to measure the 3D surface of the eye 342, as is known in the machine vision industry. In particular, we will describe below an example in which a plurality of images can be captured over time and processed in order to provide sufficient information for the controller to determine the distance between the HOE 341 and the eye 342.

In these examples, the camera 339 captures a plurality of images of the eye 342 as reflected by the one or more specularly reflective regions of the holographic optical element, at a respective plurality of different instants in time. That is, a sequence of images of the eye 342 can be captured by the camera 338 over time. The controller can then, for each of the plurality of captured images: process the captured image of the eye in order to recognise a pattern in the image; and compare: i) the predetermined pattern, with ii) the recognised pattern in the captured image, in order to extract a portion of the captured image in which the recognised pattern is present. The portion of the captured image can be a subset of the captured image that is defined by a bounding box with a predefined size that includes the recognised pattern, for example. The controller can then combine the extracted portions of the captured images to provide a combined-image of the eye including a plurality of recognised patterns. Such extracted portions can be stitched together in any way that is known in the art. The controller can then compare each of the plurality of recognised patterns in the combined-image with the predetermined pattern in order to determine a distance between: i) the regions of the eye that are represented by the recognised patterns; and ii) the holographic optical element. In one example, the predetermined pattern is a 1-dimensional line, which will be imaged on a 2-dimensional sensor. An offset on the 2-dimensional sensor represents a measure of distance between the HOE and the eye, which can be calculated by triangulation of the emitted line and the detected line using the known base distance between the projector (illuminator) and the detector (camera).

In such an example, the predetermined pattern can comprise a substantially one-dimensional pattern. That is, it can be a straight line that is very thin, and therefore has a negligible width when compared with its length. Such a predetermined pattern can provide sufficient information for corneal topography mapping to be performed if the user moves their eye during image capture such that the recognised pattern is present on different regions of the user's eye in the different images. In this way, the straight line (1-dimensional pattern) can be considered as scanning the eye over time (i.e., a temporal scan) such that the distance to different regions of the eye can be determined over time. Then, those distances can be combined in order to generate the 3-dimenionsal topographical map of the eye. It will be appreciated that this processing can be performed with a 2-dimensional predetermined pattern too, of course.

Returning to Figure 5, in addition to the shapes of the diffusely reflective regions of the HOE that are reflected as images on the cornea (concentric rings) that are visible in the image, three glints 550 are visible on each side of the pupil. This is because the illuminators are co-located with the cameras, and therefore they illuminate the eye via the HOE.

Figure 6 shows another example of an image of an eye that has been captured by a camera via an HOE with a concentric ring pattern of specularly and diffusely reflective regions. In this example, the eye is directly illuminated (not via the HOE), and therefore no glints are present in the image.

Returning to Figure 5, as discussed above, the specularly reflective areas of the HOE have been divided into several discrete areas. In some examples, these different areas can have different properties. For instance, they can be specularly reflective for different wavelengths of light and / or can have different polarisations. In addition, they can be side-by-side in the HOE, and / or they can be overlaid with each other in different films / different layers of the HOE. Such examples can be considered as multiplexed holograms because they can be used to obtain multiple images of the same eye from a single HOE and a single camera.

As shown in Figure 5, the discrete specularly reflective areas can have blank spaces in between them. In this way, as discussed above, the diffuse areas (between the specularly reflective areas) each reflect an "image" on the cornea. By having several areas, discrete shapes are present in the captured image of the eye that can be used to extract information of the eye. Such information: can include a distance to the cornea surface (as discussed at length above); can include eyeball centre; can help with glint detection and matching; and can be used for measuring the corneal surface topology.

The glints 550 can be used to find the cornea centre through known PCCR eye-tracking, while the concentric rings can be used to measure the corneal topography through their distortion and relative placement (as discussed above). It is noticeable from Figure 5 that the concentric rings are much less bright than the glints. This is an advantage for glint detection, and is due to the patterned light being spread out over a much larger surface as it is reflected by the diffusely reflective regions of the HOE.

Moving on to Figure 6, the image shows a cornea reflecting a concentric ring pattern in the same way as Figure 5. The centre of the rings can be used for PCCR eye-tracking, and the rings can be used to map corneal topography. Beneficially, the centre of the ring pattern can be found even if only a part of the ring pattern is visible. Standard circle Hough transforms can be used. A benefit of using a concentric ring pattern, as opposed to a glint pattern, is that there is only one centre instead of multiple glint centres. It also avoids a need to keep track of individual glints. Mapping out the corneal topography can be done during regular personal calibration. The corneal topography can be used later for more accurate cornea centre and pupil mapping as part of a subsequent eye-tracking operation. This therefore advantageously can account for the variability between the corneas of individual users.

Eye-tracking operations can utilise an eye-tracking model, which typically assumes a spherical cornea. However, real corneas are ellipsoidal with a radius of curvature that is larger at the edge of the cornea and is smaller in the centre. For instance, a typical radius of curvature at the centre of the cornea can be 7.8 mm and 10 -11 mm at the edge. The radius of curvature error can result in an error in distance calculation to the eye from the camera. Further details can be found in: "General Theory of Remote Gaze Estimation Using the Pupil Center and Corneal Reflections" Guestring and Eisenman IEEE TRANSACTIONS ON BIOMEDICAL ENGINEERING, VOL. 53, NO. 6, JUNE 2006 pp. 1124-1133.

Any of the controllers described herein can use the determined distance from the holographic optical element to the eye (or the plurality of determined distances, which can be represented as a corneal topography map) as part of a subsequent eye-tracking operation. Advantageously, use of the determined distances can improve the accuracy, reliability and / or robustness of the subsequent eye-tracking operation. In one example, the controller can update an eye-tracking model based on the determined distance from the holographic optical element to the eye and / or a determined corneal topography map. In this way, the eye-tracking model can represent the determined corneal topography map instead of assuming that the cornea has a spherical profile. The controller can then use the updated eye-tracking model to perform eye-tracking based on subsequently captured images of the eye.

With any of the hologram-based eye-tracking systems described herein, the illuminators can be provided as vertical-cavity surface-emitting lasers (VCSELs). VCSELs can be well-suited to these applications due to bandwidth limitations of holograms. One or two VCSELs can be used, in some examples, to provide 2 or 4 glints depending on whether single or double holograms are used (i.e., HOEs that include one or two virtual mirrors). At large gaze angles, the glints can fall off. For an LED based eye-tracking system, 8-10 LEDs can be integrated into a head-mounted device. To keep costs down, it can be beneficial to use only 1-2 VCSELs per eye. By using a concentric ring pattern that covers the whole eye (as shown in Figures 5 and 6), the pattern can be always present, even at large angles. If concentric rings are used, the centre can be the reference point for gaze angles. Moreover, the concentric rings can be used for cornea-topography using keratometer principles.

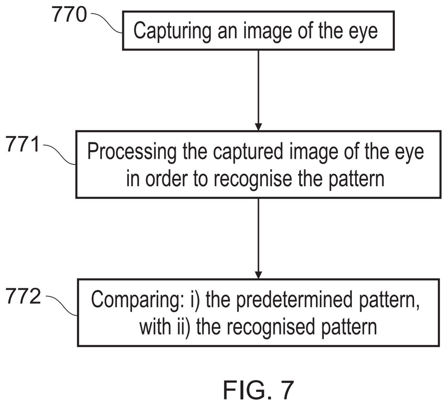

Figure 7 shows schematically a computer implemented method of operating an eye-tracking system according to the present disclosure. As discussed in detail above, the eye-tracking system includes at least a holographic optical element that comprises one or more specularly reflective regions and one or more diffusely reflective regions. The one or more specularly reflective regions and the one or more diffusely reflective regions of the holographic optical element are provided as a predetermined pattern.

At step 770, the method includes capturing an image of the eye as reflected by the one or more specularly reflective regions of the holographic optical element, such that a pattern that is defined by the one or more diffusely reflective regions of the holographic optical element is visible on the eye in the captured image.

At step 771, the method includes processing the captured image of the eye in order to recognise the pattern of the one or more diffusely reflective regions in the image. Then, at step 772, the method includes comparing: i) the predetermined pattern, with ii) the recognised pattern in the captured image, in order to determine a distance from the holographic optical element to the eye. Various specific implementations of how such a distance can be determined are described above.

It will be appreciated for the above description that various of the examples disclosed herein include an illuminator that can both: provide background illumination of the eye by illuminating the one or more diffusely reflective regions of the HOE; and provide one or more "glints" on the eye by illuminating the one or more specularly reflective regions of the HOE. Furthermore, the HOE can include a plurality of specularly reflective regions, such that the illuminator provides a plurality of "glints" on the eye by illuminating the plurality of specularly reflective regions of the HOE. As will be appreciated from this description, the plurality of glints can be achieved by using a plurality of different specularly reflective regions that define virtual mirrors at different angles, a plurality of different specularly reflective regions for different wavelengths, or a plurality of different polarisations, for example. Furthermore, the illuminator can illuminate the eye via the one or more specularly reflective regions and the one or more diffusely reflective regions of the holographic optical element. It is not necessary for the diffusely reflective regions of the holographic optical element to be in the field of view of the camera of the eye-tracking system.

## Claims

1. An eye-tracking system for tracking an eye, the eye-tracking system comprising:
a holographic optical element, which comprises one or more specularly reflective regions and one or more diffusely reflective regions, wherein the one or more specularly reflective regions and the one or more diffusely reflective regions of the holographic optical element are provided as a predetermined pattern;
a camera that is configured to capture an image of the eye as reflected by the one or more specularly reflective regions of the holographic optical element, such that a pattern that is defined by the one or more diffusely reflective regions of the holographic optical element is visible on the eye in the captured image; and
a controller that is configured to:
process the captured image of the eye in order to recognise the pattern of the one or more diffusely reflective regions in the image; and
compare: i) the predetermined pattern, with ii) the recognised pattern in the captured image, in order to determine a distance from the holographic optical element to the eye.

2. The eye-tracking system of claim 1, wherein the controller is configured to determine a plurality of distances from the holographic optical element to respective different regions of the eye.

3. The eye-tracking system of claim 2, wherein the controller is configured to determine a corneal topography map based on the determined plurality of distances.

4. The eye-tracking system of any preceding claim, wherein the predetermined pattern comprises a 2-dimensional pattern with a predetermined spacing between features in the pattern in two perpendicular dimensions.

5. The eye-tracking system of claim 4, wherein the controller is configured to:
compare: i) the predetermined spacing between features in the predetermined pattern, with ii) a recognised spacing between corresponding features in the recognised pattern in the captured image in order to:
determine a distance from the holographic optical element to regions of the eye on which the corresponding features in the recognised pattern in the captured image are present.

6. The eye-tracking system of any preceding claim, wherein the controller is configured to:
compare: i) the predetermined pattern, with ii) the recognised pattern in the captured image, in order to:
detect any distortions in the recognised pattern; and
determine the distance from the holographic optical element to the eye based on the detected distortions.

7. The eye-tracking system of any preceding claim, wherein:
the camera is configured to capture a plurality of images of the eye as reflected by the one or more specularly reflective regions of the holographic optical element, at a respective plurality of difference instants in time, such that a pattern that is defined by the one or more diffusely reflective regions of the holographic optical element is visible on the eye in each of the captured images; and
the controller is configured to:
for each of the plurality of captured images:
process the captured image of the eye in order to recognise the pattern in the image of the one or more diffusely reflective regions; and
compare: i) the predetermined pattern, with ii) the recognised pattern in the captured image, in order to extract a portion of the captured image in which the recognised pattern is present;
combine the extracted portions of the captured images to provide a combined-image of the eye including a plurality of recognised patterns; and
compare each of the plurality of recognised patterns in the combined-image with the predetermined pattern in order to determine a distance between: i) the regions of the eye that are represented by the recognised patterns; and ii) the holographic optical element.

8. The eye-tracking system of claim 7, wherein the predetermined pattern comprises a substantially one-dimensional pattern.

9. The eye-tracking system of any preceding claim, wherein the controller is further configured to:
update an eye-tracking model based on the determined distance from the holographic optical element to the eye; and
use the updated eye-tracking model to perform eye-tracking based on subsequently captured images of the eye.

10. The eye-tracking system of claim 9, wherein, during a subsequent eye-tracking operation, the illuminator is configured to:
provide background illumination of the eye by illuminating the one or more diffusely reflective regions of the holographical optical element; and
provide one or more "glints" on the eye by illuminating the one or more specularly reflective regions of the holographical optical element.

11. The eye-tracking system of claim 10, wherein the holographic optical element comprises a plurality of specularly reflective regions, such that the illuminator is configured to provide a plurality of "glints" on the eye by illuminating the plurality of specularly reflective regions of the holographical optical element.

12. The eye-tracking system of any preceding claim, wherein the eye-tracking system comprises a head-mounted device.

13. The eye-tracking system of claim 12, wherein the head-mounted device comprises a pair of glasses having lens regions and a pair of arms, and wherein:
the camera is mounted to one of the arms; and
the holographic optical element is located over at least one of the lens regions.

14. The eye-tracking system of claim 13, further comprising an illuminator that is configured to illuminate the eye via the one or more specularly reflective regions and the one or more diffusely reflective regions of the holographic optical element.

15. A computer implemented method of operating an eye-tracking system, wherein the eye-tracking system comprises:
a holographic optical element, which comprises one or more specularly reflective regions and one or more diffusely reflective regions, wherein the one or more specularly reflective regions and the one or more diffusely reflective regions of the holographic optical element are provided as a predetermined pattern;
the method comprising:
capturing an image of the eye as reflected by the one or more specularly reflective regions of the holographic optical element, such that a pattern that is defined by the one or more diffusely reflective regions of the holographic optical element is visible on the eye in the captured image; and
processing the captured image of the eye in order to recognise the pattern of the one or more diffusely reflective regions in the image; and
comparing: i) the predetermined pattern, with ii) the recognised pattern in the captured image, in order to determine a distance from the holographic optical element to the eye.
